# EUROPEAN PATENT SPECIFICATION

(11) **EP 4 188 905 B1**
(45) Date of publication and mention of the grant of the patent: **11.09.2024**
(21) Application number: 21752516.1
(22) Date of filing: 26.07.2021
(51) Int. Cl.: C07C 213/10, C07C 217/08, C07C 57/38

(54) **METHOD FOR THE PURIFICATION OF VILANTEROL TRIFENATATE**
VERFAHREN ZUR REINIGUNG VON VILANTEROL-TRIFENATAT
PROCÉDÉ DE PURIFICATION DE TRIFÉNATATE DE VILANTÉROL

(30) Priority: 27.07.2020 EP 20382677
(43) Date of publication of application: 07.06.2023
(73) Proprietor: INKE, S.A., 08755 Castellbisball - Barcelona (ES)
(72) Inventor: DALMASES BARJOAN, Pere, 08755 CASTELLBISBAL (ES); CAPDEVILA URBANEJA, Enric, 08755 CASTELLBISBAL (ES); RAMÍREZ ARTERO, Jesús, 43130 TARRAGONA (ES); CERÓN BERTRAN, Jordi Carles, 43761 LA POBLA DE MONTORNÈS (ES)
(74) Representative: ZBM Patents - Zea, Barlocci & Markvardsen
(86) International application number: PCT/EP2021/070903
(87) International publication number: WO 2022/023291

(56) References cited:
- WO-A1-03/024439
- WO-A2-2014/041565

## Description

This application claims the benefit of European Patent Application EP20382677.1 filed on July 27th, 2020

### Technical Field

The present invention relates to a process for the purification of vilanterol trifenatate.

### Background Art

Vilanterol trifenatate is the generic name of compound (R)-4-(2-((6-(2-((2,6-dichlorobenzyl)oxy)ethoxy)hexyl)amino)-1-hydroxyethyl)-2-(hydroxymethyl)phenol 2,2,2-triphenylacetate, having the following chemical structure:

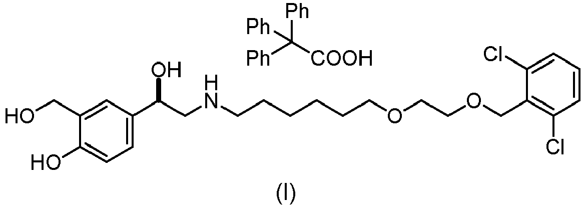

Vilanterol trifenatate is a selective long-acting beta₂-adrenergic agonist. It is administered by inhalation as a dry powder formulation in combination with umeclidinium bromide and/or fluticasone furoate for the treatment of chronic obstructive pulmonary disease (COPD) and asthma.

Vilanterol trifenatate was first disclosed in document WO 2003/024439, which discloses a process wherein vilanterol trifenatate is crystallized in ethanol. Nevertheless, the product is obtained with an undesirable high impurity level.

Document WO 2014/041565 discloses crystallization of vilanterol trifenatate in acetone, which results in a decreased impurity level, at the expense of a significantly low yield. Additionally, acetone is highly flammable, highly reactive and harmful to human health and, thus, it is not a convenient solvent for industrial applicability.

Document WO 2017/001907 discloses a process for the preparation of vilanterol trifenatate, wherein vilanterol tartrate is converted in a multi-step process via the base (by addition of the corresponding acid) to the trifenatate salt. Allegedly, the process allows obtaining a product with a relatively high purity, particularly with a low amount of impurity

A of the following formula:

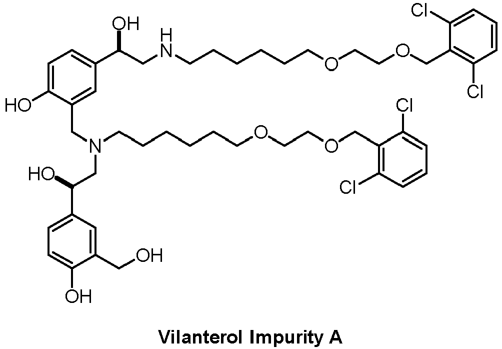

However, the process is carried out in a mixture of DCM, MTBE, and EtOH, and requires a laborious workup.

Therefore, there is still the need of finding new methods that allow preparing vilanterol trifenatate in good yields and purity and, at the same time, which are of easy industrial applicability.

### Summary of Invention

Inventors have found a new process for the purification of vilanterol trifenatate that overcomes the drawbacks of the processes disclosed in the prior art. Surprisingly, they have found that by crystallizing vilanterol trifenatate from some specific ketone solvents, a solid product is obtained in both good yields and high purity.

Thus, an aspect of the present disclosure refers to a method for the purification of vilanterol trifenatate of formula (I) 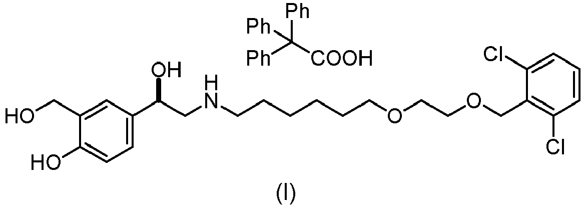 comprising crystallizing vilanterol trifenatate from a ketone solvent selected from the group consisting of methyl ethyl ketone (MEK), methyl isobutyl ketone (MIK), ethyl isopropyl ketone, methyl isopropyl ketone, 3-methyl-2-pentanone, and a mixture thereof.

### Brief Description of Drawings

Fig. 1 shows the SEM images of crystals of vilanterol trifenatate obtained by Ostwald ripening from MEK at 2550 magnification.
Fig. 2 shows the SEM images of crystals of vilanterol trifenatate obtained by Ostwald ripening from MIK at 2500 magnification.
Fig. 3 shows the SEM images of crystals of vilanterol trifenatate obtained by Ostwald ripening from ethanol at 2500 magnification.

### Detailed description of the invention

All the terms used in the present description, unless otherwise indicated, are to be understood in their common meaning as known in the art.

The term "V" preceded by a number means how many times in terms of volume the amount of a substance exceeds the given amount of another substance in terms of weight. For example, given 7.5 g of vilanterol trifenatate, adding 8V of MEK means to add 60 mL of MEK.

The term "seeding" refers to the addition of a crystalline material to facilitate crystallization. In the context of the present disclosure, seeding is carried out with crystals of vilanterol trifenatate.

The term "Ostwald ripening" refers to the growth of larger crystals from those of smaller size, which have a higher solubility than the larger ones by temperature cycling. In the process, many small crystals formed initially slowly disappear, except for a few that grow larger, at the expense of the small crystals. The smaller crystals act as fuel for the growth of bigger crystals. Ostwald ripening is the key process in the digestion of precipitates. The digested precipitate is generally purer, more homogeneous and easier to wash and filter.

The term "room temperature" refers to about 20 °C -25 °C.

The term "about" comprises the range of experimental error which may occur in a measurement. In particular, when referred to a value, it means the given value plus or minus 5% and, when referred to a range, it means the outer values plus or minus 5%.

Processes of the prior art attempt to obtain vilanterol trifenatate with the highest purity as possible. Particularly, there is interest in providing a process that allows minimizing the presence of impurity A mentioned above in the final product. The inventors realized that the mentioned impurity proved to be difficult to remove, and even the subsequent recrystallization from the solvents disclosed in the prior art for this purpose did not allow obtaining the final product with the desired purity level and physical properties.

After extensive experimentation, the inventors have surprisingly found that crystallization of vilanterol trifenatate from methyl ethyl ketone (MEK), methyl isobutyl ketone (MIK), ethyl isopropyl ketone, methyl isopropyl ketone, 3-methyl-2-pentanone, or a mixture thereof, allows achieving the mentioned aims.

Thus, an aspect of the present disclosure refers to a method for the purification of vilanterol trifenatate comprising crystallizing vilanterol trifenatate from at least one of the mentioned ketone solvents. Particularly, the ketone solvent is selected from MEK, MIK, or a mixture thereof.

In a particular embodiment, the method of purification is carried out in MEK as the ketone solvent.

In another particular embodiment, the method of purification is carried out in MIK as the ketone solvent.

In an embodiment, optionally in combination with one or more features of the particular embodiments defined above, the method for the purification of vilanterol trifenatate comprises:
a) providing a solution of vilanterol trifenatate in an appropriate amount of the ketone solvent at a temperature lower than the boiling point of the solvent such as from 55 °C to 70 °C;
b) cooling down the solution to a temperature from room temperature to 0 °C, particularly to room temperature, to crystallize vilanterol trifenatate; and
c) isolating the crystallized solid.

The term "appropriate amount" of the ketone solvent relates to the amount of the ketone solvent needed so that vilanterol trifenatate crystallizes when cooling of step b) is carried out. Particularly, the amount of the ketone solvent is such that vilanterol trifenatate is solved at its maximum concentration in the particular solvent, i.e. the solution is a saturated solution of vilanterol trifenatate, at the mentioned temperature.

Particularly, the method for the purification of vilanterol trifenatate in MEK comprises:
a) providing a solution of vilanterol trifenatate in MEK, such as in from about 8V to 10V of MEK at a temperature from 55 °C to 70 °C;
b) cooling the solution at room temperature to crystallize vilanterol trifenatate; and
c) isolating the crystallized solid.

Particularly, the method for the purification of vilanterol trifenatate in MIK comprises:
a) providing a solution of vilanterol trifenatate in MIK, such as in from about 30V to about 35V of MIK at a temperature from 55 °C to 70 °C;
b) cooling the solution at room temperature to crystallize vilanterol trifenatate; and
c) isolating the crystallized solid.

A way to improve the crystallization process of the present invention, in particular of promoting crystallization in a controlled way, is by seeding with some crystals of the product. Accordingly, in another embodiment, optionally in combination with one or more features of the particular embodiments defined above or below, the method comprises seeding with vilanterol trifenatate crystals to initiate the crystallization. Particularly, after obtaining the solution of vilanterol trifenatate, the solution is cooled down to a temperature higher than room temperature, such as from 45 °C to 60 °C, then seeded with vilanterol trifenatate crystals, and cooled down to a temperature from room temperature to 0 °C, particularly to room temperature, to crystallize vilanterol trifenatate.

Isolation of vilanterol trifenatate crystals obtained according to the method of the present disclosure can be carried out according to methods known in the art, including, without being limited to, filtration, particularly filtration under vacuum. Then, the crystalline solid can be washed with the crystallization solvent at a temperature from room temperature to 0 °C, particularly at room temperature, and dried at a temperature from 50 °C to 60 °C for a suitable time period until constant weight. A suitable time period can be, for example, from 10 to 20 hours, such as about 16 hours. The drying can be carried out according to methods known in the art including, but not limited to, drying under reduced pressure.

In a more particular embodiment, optionally in combination with one or more features of the particular embodiments defined above or below, the process comprises a previous step comprising adding triphenylacetic acid to a solution of vilanterol free base in the ketone solvent to form the solution of vilanterol trifenatate in the ketone solvent. Vilanterol free base can be obtained by any method known in the art, particularly a method wherein impurity A is formed.

In a more particular embodiment, optionally in combination with one or more features of the particular embodiments defined above or below, the process comprises a previous step comprising mixing vilanterol trifenatate with an appropriate amount of the ketone solvent and heating the mixture until dissolution.

In another particular embodiment of the process of the present disclosure, optionally in combination with one or more features of the particular embodiments defined above or below, the solution of the vilanterol trifenatate is a saturated solution.

In another embodiment, optionally in combination with one or more features of the particular embodiments defined above or below, the method of purification of vilanterol trifenatate further comprises recrystallizing vilanterol trifenatate from the same or a different solvent, the solvent being a ketone solvent selected from the group consisting of methyl ethyl ketone (MEK), methyl isobutyl ketone (MIK), ethyl isopropyl ketone, methyl isopropyl ketone, 3-methyl-2-pentanone, and a mixture thereof.

Said purification by recrystallization can be carried out according to methods known in the art, in particular, by dissolving vilanterol trifenatate at warm in at least one of the mentioned ketone solvents, and then cooling the resulting solution to precipitate the product. In a more particular embodiment, optionally in combination with one or more features of the particular embodiments defined above or below, the method comprises seeding with vilanterol trifenatate crystals to initiate the recrystallization. Vilanterol trifenatate crystals used for seeding can be obtained by any method known in the art, particularly by the crystallization and/or recrystallization methods of the present disclosure.

In a particular embodiment, recrystallization is carried out in MEK.

Particularly, the recrystallization of vilanterol trifenatate in MEK comprises:
a) suspending vilanterol trifenatate in MEK, such as in from about 8V to 10V of MEK;
b) heating the suspension such as at a temperature from 55 °C to 60 °C until a solution is obtained;
c) cooling the solution at a temperature from 46 °C to 55 °C;
d) seeding the solution with vilanterol trifenatate crystals;
e) cooling the mixture at a temperature from 20 °C to 25 °C to crystallize vilanterol trifenatate; and
f) isolating the crystallized solid.

In another particular embodiment, recrystallization is carried out in MIK as the ketone solvent.

Particularly, the recrystallization of vilanterol trifenatate in MIK comprises:
a) suspending vilanterol trifenatate in MIK, such as in from about 30V to about 35V of MIK;
b) heating the suspension such as at a temperature from 65 °C to 70 °C until a solution is obtained;
c) cooling the solution at a temperature from 54 °C to 62 °C;
d) seeding the solution with vilanterol trifenatate crystals;
e) cooling the mixture at a temperature from 20 °C to 25 °C to crystallize vilanterol trifenatate; and
f) isolating the crystallized solid.

Additionally, in an attempt to further increase the purity of the final product, the inventors realized that by carrying out recrystallization of vilanterol trifenatate by Ostwald ripening using the mentioned ketone solvents, no aggregates are formed, as opposed to other solvents such as ethanol. Besides, the size of the crystals that are generated from the mentioned ketone solvents is more convenient when micronizing than the ones generated from ethanol.

Thus, in another embodiment, optionally in combination with one or more features of the particular embodiments defined above or below, recrystallization is carried out by Ostwald ripening. Particularly, Ostwald ripening comprises:
a) providing a suspension of vilanterol trifenatate in the ketone solvent;
b) heating the suspension of step a) until a solution is obtained;
c) cooling the solution to a first temperature above room temperature;
d) seeding the solution with vilanterol trifenatate crystals to give a suspension;
e) cooling the suspension to a second temperature between the first temperature and room temperature while stirring; heating the suspension to the first temperature while stirring; and repeating step e) at least one more time;
f) cooling the suspension at a temperature from 20 °C to 25 °C; and
g) isolating vilanterol trifenatate crystals from the suspension of step f) at room temperature.

In a particular embodiment, Ostwald ripening is carried out in MEK and the method comprises the following steps:
a) providing a suspension of vilanterol trifenatate in MEK;
b) heating the suspension of step a) until a solution is obtained;
c) cooling the solution to 46 °C-52 °C, preferably to 49 °C;
d) seeding the solution with vilanterol trifenatate crystals to give a suspension;
e) cooling the suspension to 37 °C-43 °C, preferably to 40 °C, while stirring at this temperature for 10-30 min, preferably for 15 min;
   heating the suspension to 46 °C -52 °C, preferably to 49 °C, while stirring at this temperature for 10-30 min, preferably for 15 min;
   cooling the suspension to 37 °C-43 °C, preferably to 40 °C, while stirring at this temperature for 10-30 min, preferably for 15 min;
   heating the suspension to 46 °C -52 °C, preferably to 49 °C, while stirring at this temperature for 10-30 min, preferably for 15 min; and
f) cooling the suspension at a temperature from 20 °C to 25 °C; and
g) isolating vilanterol trifenatate crystals from the suspension of step f).

In another particular embodiment, Ostwald ripening is carried out in MIK and the method comprises the following steps:
a) providing a suspension of vilanterol trifenatate in MIK;
b) heating the suspension of step a) until a solution is obtained;
c) cooling the solution to 54 °C-60 °C, preferably to 57 °C;
d) seeding the solution with vilanterol trifenatate crystals to give a suspension;
e) cooling the suspension to 44 °C-50 °C, preferably to 47 °C, while stirring at this temperature for 10-30 min, preferably for 15 min;
   heating the suspension to 54 °C -60 °C, preferably to 57 °C, while stirring at this temperature for 10-30 min, preferably for 15 min;
   cooling the suspension to 44 °C-50 °C, preferably to 47 °C, while stirring at this temperature for 10-30 min, preferably for 15 min;
   heating the suspension to 54 °C-60 °C, preferably to 57 °C, while stirring at this temperature for 10-30 min, preferably for 15 min;
f) cooling the suspension at a temperature from 20 °C to 25 °C; and
g) isolating vilanterol trifenatate crystals from the suspension of step f).

Throughout the description and claims the word "comprise" and variations of the word, are not intended to exclude other technical features, additives, components, or steps. Furthermore, the word "comprise" encompasses the case of "consisting of".

The following examples and drawings are provided by way of illustration, and they are not intended to be limiting of the present invention. Furthermore, the present invention covers all possible combinations of particular and preferred embodiments described herein.

### Examples

The HPLC analysis was carried out in the following column and conditions:

| **Vilanterol Trifenatate** | | | | |
|---|---|---|---|---|
| **Mobile phase** | *Mobile phase A:* | | | |
| | 1.36 g KH₂PO₄ and 3 ml of TEA into 900 mL of Water Milli-Q, adjusted to pH=2.0 ± 0.1 with H₃PO₄: H₂O (1:1). Dilute to 1000 mL with water and filter (0.45µm). | | | |
| | *Mobile phase B:* | | | |
| | ACN:IPA (9:1) | | | |

| **Chromatographic System** | | | | |
|---|---|---|---|---|
| **Mode** | LC | | | |
| **Detector** | UV - 210 nm, Bandwidth 10 nm | | | |
| | Reference wavelength 550 nm, Bandwidth 100 nm | | | |
| **Column** | *Size:* | 4.6 mm x 25 cm | | |
| | *Stationary phase:* | Octadecyl silane silica gel for chromatography (5 µm) | | |
| | *Brand name:* | Zorbax Eclipse XDB C18 | | |
| **Column Temperature** | 35 °C | | | |
| **Flow rate** | 1.5 mL/min | | | |
| **Injection Volume** | 5 µL (Use Needle Wash with MeOH) | | | |
| | *Sample Manager must be at* 5°C | | | |

| **Gradient Elution** | ***Time (min)*** | ***Mobile Phase A (per cent VIV)*** | ***Mobile Phase B (per cent V*/*V)*** | ***Comment*** |
|---|---|---|---|---|
| | 0-20 | 71 | 29 | Initial conditions |
| | 20 - 30 | 71 → 20 | 29 → 80 | Linear Gradient |
| | 30 - 45 | 20 | 80 | Isocratic |
| | 45- 45,1 | 20→ 71 | 80 → 29 | Initial conditions |
| | - System equilibration before injection: 5 min | | | |

| | | | | |
|---|---|---|---|---|
| HLPC values are %area. | | | | |

### Example 1. Preparation of vilanterol trifenatate

Vilanterol trifenatate was prepared according to the following reaction scheme:

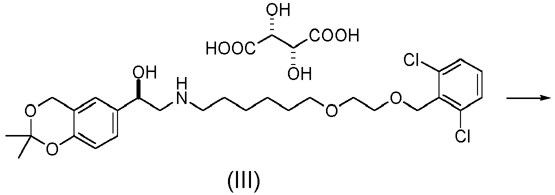

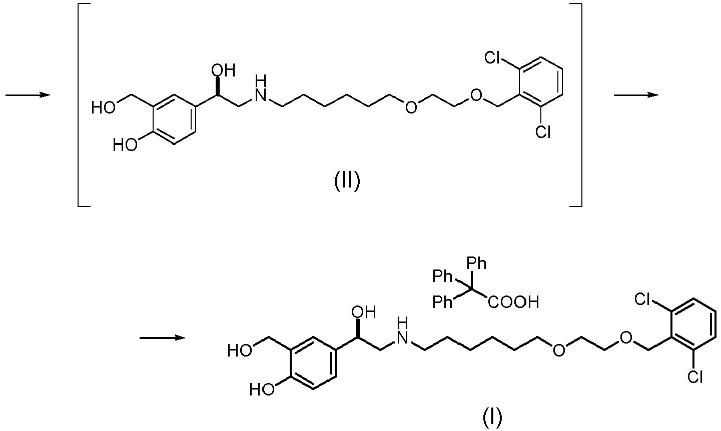

As shown below, both vilanterol base (II) in solution and crystallized vilanterol trifenatate (I) contain a non-desirable amount of impurity A.

40,0 g (59,1 mmol) of compound (III) were stirred in a mixture of 253 mL of ACN and 355 mL of HCl 0,5 N (177,4 mmol, 3,0 eq.) at 4 °C for 48 h. Then, 344 mL of methylene chloride and 200 mL of K₂CO₃ 20% were added and phases were separated. The aqueous phase was extracted with 130 mL of methylene chloride.

The resulting organic phase, comprising compound (II) (HPLC=98,44%) and impurity A (HPLC=0,45%), was divided into 4 equal parts (parts from 1 to 4). Each part was used in Examples 2 and 3, and Comparative Examples 1 and 2 below.

### Example 2. Crystallization of vilanterol trifenatate from MEK

The solvent of part 1 obtained in Example 1 was distilled off at reduced pressure and swapped with methyl ethyl ketone (MEK). The obtained residue (compound **(II)**) was dissolved in 88 mL of MEK. 4,3 g (14,8 mmol, 1,0 eq) of solid triphenylacetic acid were added all at once. The solution was heated to 55 °C and cooled to 20-25 °C for 3 h. The white solid obtained was filtered and washed twice with 11 mL of MEK. The white solid was dried under reduced pressure at 20-25 °C for 16 h and under reduced pressure at 55 °C for 1 h. 9,2 g (11,9 mmol) of vilanterol trifenatate (I) were obtained (yield=80%) (HPLC=99,38%, Impurity A=0,19%).

### Example 3. Crystallization of vilanterol trifenatate from MIK

The solvent of part 2 obtained in Example 1 was distilled off at reduced pressure and swapped with methyl isobutyl ketone (MIK). The obtained residue (compound (II)) was dissolved in 88 mL of MIK. 4,3 g (14,8 mmol, 1,0 eq) of solid triphenylacetic acid were added all at once. The solution was heated to 55 °C and cooled to 20-25 °C for 3 h. The white solid obtained was filtered and washed twice with 11 mL of MIK. The white solid was dried under reduced pressure at 20-25 °C for 16 h and under reduced pressure at 55 °C for 1 h. 10,4 g (13,4 mmol) of vilanterol trifenatate (I) were obtained (yield=91%) (HPLC=99,28%, Impurity A=0,23%).

### Comparative Example 1. Crystallization of vilanterol trifenatate from acetone

The solvent of part 3 obtained in Example 1 was distilled off at reduced pressure and swapped with acetone. The obtained residue (compound (II)) was dissolved in 88 mL of acetone. 4,3 g (14,8 mmol, 1,0 eq) of solid triphenylacetic acid were added all at once. The solution was heated to 55 °C and cooled to 20-25 °C for 3 h. The white solid obtained was filtered and washed twice with 11 mL of acetone. The white solid was dried under reduced pressure at 20-25 °C for 16 h and under reduced pressure at 55 °C for 1 h. 7,1 g (9,2 mmol) of vilanterol trifenatate (I) were obtained (yield=62%) (HPLC=99,47%, Impurity A=0,14%).

### Comparative Example 2. Crystallization of vilanterol trifenatate from ethanol

The solvent of part 4 obtained in Example 1 was distilled off at reduced pressure and swapped with ethanol. The obtained residue (compound (II)) was dissolved in 88 mL of ethanol. 4,3 g (14,8 mmol, 1,0 eq) of solid triphenylacetic acid were added all at once. The solution was heated to 55 °C and cooled to 20-25 °C for 3 h. The white solid obtained was filtered and washed twice with 11 mL of ethanol. The white solid was dried under reduced pressure at 20-25 °C for 16 h and under reduced pressure at 55 °C for 1 h. 9,3 g (12,0 mmol) of vilanterol trifenatate (I) were obtained (yield=81%) (HPLC=99,11%, Impurity A=0,28%).

### Example 4. Crystallization of vilanterol trifenatate from MEK

Vilanterol trifenalate was obtained following Example 2. After being filtered and washed with MEK, the white solid was dried under reduced pressure at 20-25 °C for 16 h and under reduced pressure at 55 °C for 1 h. 36,3 g (46,8 mmol) of vilanterol trifenatate (I) were obtained (yield=79%) (HPLC=99,37%, Impurity A=0,18%).

### Example 5. Recrystallization vilanterol trifenatate in MEK

9,0 g (11,6 mmol) of vilanterol trifenatate obtained in Example 4 were stirred at 20-25 °C with 72 mL of MEK. The suspension was heated at 55-60 °C until a solution was obtained. The solution was cooled to 50-55 °C, seeded with vilanterol trifenatate crystals and cooled to 20-25 °C for 3 h. The white solid obtained was filtered and washed twice with 9 mL of MEK. The white solid was dried under reduced pressure at 55 °C for 16 h. 8,1 g (10,5 mmol) of vilanterol trifenatate (I) were obtained (yield= 90%) (HPLC=99,60%, Impurity A=0,08%).

### Example 6. Recrystallization vilanterol trifenatate in MIK

9,0 g (11,6 mmol) of vilanterol trifenatate obtained in Example 4 were stirred at 20-25 °C with 270 mL of MIK. The suspension was heated at 65-67 °C until a solution is obtained. The solution was cooled to 60-62 °C, seeded with vilanterol trifenatate crystals and cooled to 20-25 °C for 3 h. The white solid obtained was filtered and washed twice with 9 mL of MIK. The white solid was dried under reduced pressure at 55 °C for 16 h. 7,8 g (10,1 mmol) of vilanterol trifenatate (I) were obtained (yield= 87%) (HPLC=99,54%, Impurity A=0,09%).

### Comparative Example 3. Recrystallization vilanterol trifenatate in acetone

9,0 g (11,6 mmol) of vilanterol trifenatate obtained in Example 4 were stirred at 20-25 °C with 90 mL of acetone. The suspension was heated at 55-56 °C until a solution was obtained. The solution was cooled to 50-52 °C, seeded with vilanterol trifenatate crystals and cooled to 20-25 °C for 3 h. The white solid obtained was filtered and washed twice with 9 mL of acetone. The white solid was dried under reduced pressure at 55 °C for 16 h. 5,5 g (7,1 mmol) of vilanterol trifenatate (I) were obtained (yield= 61%) (HPLC=99,57%, Impurity A=0,09%).

### Comparative Example 4. Recrystallization vilanterol trifenatate in ethanol

9,0 g (11,6 mmol) of vilanterol trifenatate obtained in Example 4 were stirred at 20-25 °C with 90 mL of ethanol. The suspension was heated at 55-60 °C until a solution is obtained. The solution was cooled to 50-55 °C, seeded with vilanterol trifenatate crystals and cooled to 20-25 °C for 3 h. The white solid obtained was filtered and washed twice with 9 mL of ethanol. The white solid was dried under reduced pressure at 55 °C for 16 h. 7,9 g (10,2 mmol) of vilanterol trifenatate (I) were obtained (yield= 88%) (HPLC=99,44%, Impurity A=0,17%).

### Example 7. Ostwald ripening experiment with MEK

Vilanterol trifenatate (100 g, 129 mmol) was suspended in MEK (850 mL). The suspension was heated to 55 °C until a solution was observed. Then, the system was cooled to 49°C, seeded with vilanterol trifenatate crystals and the following Ostwald ripening process was then followed:
1. the suspension was cooled to 40 °C and stirred at this temperature for 15 min;
2. the suspension was heated to 49 °C and stirred at this temperature for 15 min;
3. the suspension was cooled to 40 °C and stirred at this temperature for 15 min; and
4. the suspension was heated to 49 °C and stirred at this temperature for 15 min.

The obtained suspension was cooled to 20 °C and stirred at this temperature for 1 h. The white solid obtained was filtered and dried under reduced pressure at 55 °C for 24 h. 88 g (114 mmol) of vilanterol trifenatate (I) were obtained (yield= 88%).

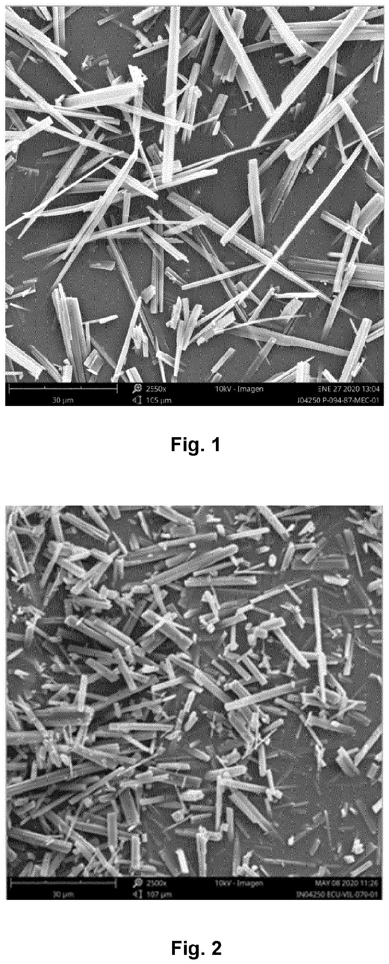

A SEM image of the crystals obtained is shown in Fig. 1, where it can be seen that no aggregates are formed with MEK.

### Example 8. Ostwald ripening experiment with MIK

Vilanterol trifenatate (20 g, 25,8 mmol) was suspended in MIK (600 mL). The suspension was heated to 65-68 °C until a solution was observed. Then, the system was cooled to 57°C, seeded with vilanterol trifenatate crystals and the following Ostwald ripening process was then followed:
1. the suspension was cooled to 47 °C and stirred at this temperature for 15 min;
2. the suspension was heated to 57 °C and stirred at this temperature for 15 min;
3. the suspension was cooled to 47 °C and stirred at this temperature for 15 min; and
4. the suspension was heated to 57 °C and stirred at this temperature for 15 min.

The obtained suspension was cooled to 20 °C and stirred at this temperature for 1 h. The white solid obtained was filtered and dried under reduced pressure at 55 °C for 16 h. 17 g (21,9 mmol) of vilanterol trifenatate (I) were obtained (yield= 85%).

A SEM image of the crystals obtained is shown in Fig. 2, where it can be seen that no aggregates are formed with MIK.

### Comparative Example 5. Ostwald ripening experiment with Ethanol

Vilanterol trifenatate (20 g, 25,8 mmol) was suspended in ethanol (210 mL). The suspension was heated to 55-56 °C until a solution was observed. Then, the system was cooled to 49 °C, seeded with vilanterol trifenatate crystals and the following Ostwald ripening process was then followed:
1. the suspension was cooled to 37 °C and stirred at this temperature for 15 min;
2. the suspension was heated to 47 °C and stirred at this temperature for 15 min;
3. the suspension was cooled to 37 °C and stirred at this temperature for 15 min; and
4. the suspension was heated to 47 °C and stirred at this temperature for 15 min.

The obtained suspension was cooled to 10 °C and stirred at this temperature for 1 h. The white solid obtained was filtered and dried under reduced pressure at 55 °C for 16 h. 18 g (23,2 mmol) of vilanterol trifenatate (I) were obtained (yield= 90%).

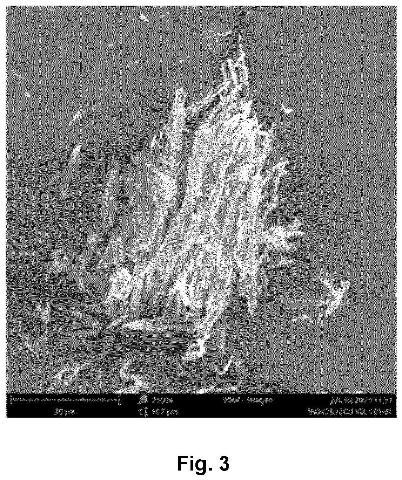

A SEM image of the crystals obtained is shown in Fig. 3, where it can be seen that aggregates are formed.

Examples and Comparative Examples above carried out by Ostwald ripening show that, advantageously, no aggregates are formed from MEK or MIK, as opposed to ethanol, and that the size of the crystals that are generated from MEK or MIK is more adequate when micronizing than from ethanol.

### Citation List

WO 2003/024439
WO 2014/041565
WO 2017/001907

## Claims

1. A method for the purification of vilanterol trifenatate of formula (I) 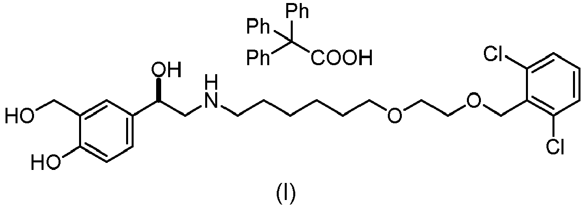 comprising a crystallization of vilanterol trifenatate from a solution of the vilanterol trifenatate in a ketone solvent selected from the group consisting of methyl ethyl ketone (MEK), methyl isobutyl ketone (MIK), ethyl isopropyl ketone, methyl isopropyl ketone, 3-methyl-2-pentanone, and a mixture thereof.

2. The method according to claim 1, wherein the crystallization comprises:
a) providing a solution of vilanterol trifenatate in an appropriate amount of the ketone solvent at a temperature lower than the boiling point of the solvent such as from 55 to 70 °C;
b) cooling down the solution to a temperature from room temperature to 0°C to crystallize vilanterol trifenatate; and
c) isolating the crystallized solid.

3. The method according to claims 1 or 2, wherein the crystallization is carried out from MEK.

4. The method according to claims 1 or 2, wherein the crystallization is carried out from MIK.

5. The method according to any one of claims 1 to 4, wherein the method comprises seeding with vilanterol trifenatate crystals to initiate the crystallization.

6. The method according to any one of claims 1 to 5, wherein the process comprises a previous step comprising adding triphenylacetic acid to a solution of vilanterol free base in the ketone solvent to form the solution of vilanterol trifenatate in the ketone solvent.

7. The method according to any one of claims 1 to 5, wherein the process comprises a previous step comprising mixing vilanterol trifenatate with an appropriate amount of the ketone solvent and heating the mixture until dissolution.

8. The method according to any one of claims 1 to 7, wherein the solution of the vilanterol trifenatate is a saturated solution.

9. The method according to any one of claims 1 to 8, further comprising recrystallizing the vilanterol trifenatate obtained in any of the claims 1-7 from a ketone solvent selected from the group consisting of methyl ethyl ketone (MEK), methyl isobutyl ketone (MIK), ethyl isopropyl ketone, methyl isopropyl ketone, 3-methyl-2-pentanone, and a mixture thereof.

10. The method according to claim 9, wherein the method comprises seeding with vilanterol trifenatate crystals to initiate the recrystallization.

11. The method according to claims 9 or 10, wherein recrystallization is carried out from MEK.

12. The method according to claims 9 or 10, wherein recrystallization is carried out from MIK.

13. The method according to claims 1 or 9, wherein crystallization or recrystallization is carried out by Ostwald ripening.

14. The method according to claim 13, wherein Ostwald ripening comprises the following steps:
a) providing a suspension of vilanterol trifenatate in the ketone solvent;
b) heating the suspension of step a) until a solution is obtained;
c) cooling the solution to a first temperature above room temperature;
d) seeding the solution with vilanterol trifenatate crystals to give a suspension;
e) cooling the suspension to a second temperature between the first temperature and room temperature while stirring; heating the suspension to the first temperature while stirring; and repeating step e) at least one more time;
f) cooling the suspension at a temperature from 20 °C to 25 °C; and
g) isolating vilanterol trifenatate crystals from the suspension of step f) at room temperature.

15. The method according to claim 14, wherein the ketone solvent is MEK or MIK.

## Patentansprüche

1. Ein Verfahren zur Reinigung von Vilanteroltrifenatat der Formel (I) 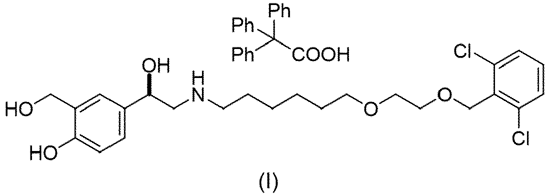 umfassend eine Kristallisation von Vilanteroltrifenatat aus einer Lösung des Vilanteroltrifenatats in einem Ketonlösungsmittel, das ausgewählt ist aus der Gruppe bestehend aus Methylethylketon (MEK), Methylisobutylketon (MIK), Ethylisopropylketon, Methylisopropylketon, 3-Methyl-2-pentanon und einer Mischung davon.

2. Das Verfahren nach Anspruch 1, wobei die Kristallisation Folgendes umfasst:
a) Bereitstellen einer Lösung von Vilanteroltrifenatat in einer geeigneten Menge des Ketonlösungsmittels bei einer Temperatur, die niedriger als der Siedepunkt des Lösungsmittels ist, wie etwa 55 bis 70 °C;
b) Abkühlen der Lösung auf eine Temperatur von Raumtemperatur bis 0 °C, um das Vilanteroltrifenat zu kristallisieren; und
c) Isolieren des kristallisierten Feststoffs.

3. Das Verfahren nach den Ansprüchen 1 oder 2, wobei die Kristallisation aus MEK durchgeführt wird.

4. Das Verfahren nach den Ansprüchen 1 oder 2, wobei die Kristallisation aus MIK durchgeführt wird.

5. Das Verfahren nach einem der Ansprüche 1 bis 4, wobei das Verfahren das Impfen mit Vilanteroltrifenatatkristallen zum Initiieren der Kristallisation umfasst.

6. Das Verfahren nach einem der Ansprüche 1 bis 5, wobei das Verfahren einen vorherigen Schritt umfasst, der das Hinzufügen von Triphenylessigsäure zu einer Lösung von freier Vilanterolbase in dem Ketonlösungsmittel umfasst, um die Lösung von Vilanteroltrifenatat in dem Ketonlösungsmittel zu bilden.

7. Das Verfahren nach einem der Ansprüche 1 bis 5, wobei das Verfahren einen vorherigen Schritt umfasst, der das Mischen von Vilanteroltrifenat mit einer geeigneten Menge des Ketonlösungsmittels und das Erhitzen der Mischung bis zur Auflösung umfasst.

8. Das Verfahren nach einem der Ansprüche 1 bis 7, wobei die Lösung des Vilanteroltrifenatats eine gesättigte Lösung ist.

9. Das Verfahren nach einem der Ansprüche 1 bis 8, ferner umfassend das Rekristallisieren des in einem der Ansprüche 1 bis 7 erhaltenen Vilanteroltrifenats aus einem Ketonlösungsmittel, das aus der Gruppe ausgewählt ist, die aus Methylethylketon (MEK), Methylisobutylketon (MIK), Ethylisopropylketon, Methylisopropylketon, 3-Methyl-2-pentanon und einer Mischung davon besteht.

10. Das Verfahren nach Anspruch 9, wobei das Verfahren das Impfen mit Vilanteroltrifenatatkristallen umfasst, um die Rekristallisation zu initiieren.

11. Das Verfahren nach den Ansprüchen 9 oder 10, wobei die Rekristallisation aus MEK durchgeführt wird.

12. Das Verfahren nach den Ansprüchen 9 oder 10, wobei die Rekristallisation aus MIK durchgeführt wird.

13. Das Verfahren nach den Ansprüchen 1 oder 9, wobei die Kristallisation oder die Rekristallisation durch Ostwald-Reifung durchgeführt wird.

14. Das Verfahren nach Anspruch 13, wobei die Ostwald-Reifung die folgenden Schritte umfasst:
a) Bereitstellen einer Suspension von Vilanteroltrifenatat in dem Ketonlösungsmittel;
b) Erhitzen der Suspension von Schritt a), bis eine Lösung erhalten wird;
c) Abkühlen der Lösung auf eine erste Temperatur oberhalb der Raumtemperatur;
d) Beimpfen der Lösung mit Vilanteroltrifenatatkristallen, um eine Suspension zu erhalten;
e) Abkühlen der Suspension auf eine zweite Temperatur zwischen der ersten Temperatur und der Raumtemperatur unter Rühren; Erwärmen der Suspension auf die erste Temperatur unter Rühren; und Wiederholen von Schritt e) mindestens einmal;
f) Abkühlen der Suspension auf eine Temperatur von 20 °C bis 25 °C; und
g) Isolieren von Vilanteroltrifenatatkristallen aus der Suspension von Schritt f) bei Raumtemperatur.

15. Das Verfahren nach Anspruch 14, wobei das Ketonlösungsmittel MEK oder MIK ist.

## Revendications

1. Un procédé pour la purification de trifénatate de vilantérol de formule (I) 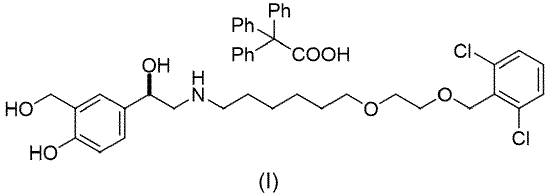 comprenant une cristallisation de trifénatate de vilanterol à partir d'une solution du trifénatate de vilanterol dans un solvant de cétone choisi dans le groupe constitué de la méthyléthylcétone (MEK), de la méthylisobutylcétone (MIK), de l'éthylisopropylcétone, de la méthylisopropylcétone, de la 3-méthyl-2-pentanone et d'un mélange de ceux-ci.

2. Le procédé selon la revendication 1, dans lequel la cristallisation comprend :
a) fournir une solution de trifénatate de vilantérol dans une quantité appropriée du solvant de cétone à une température inférieure au point d'ébullition du solvant, telle que de 55 à 70 °C ;
b) refroidir la solution à une température allant de la température ambiante à 0 °C pour cristalliser le trifénatate de vilantérol ; et
c) isoler le solide cristallisé.

3. Le procédé selon les revendications 1 ou 2, dans lequel la cristallisation est réalisée à partir de MEK.

4. Le procédé selon les revendications 1 ou 2, dans lequel la cristallisation est réalisée à partir de MIK.

5. Le procédé selon l'une quelconque des revendications 1 à 4, dans lequel le procédé comprend l'ensemencement avec des cristaux de trifénatate de vilantérol pour initier la cristallisation.

6. Le procédé selon l'une quelconque des revendications 1 à 5, dans lequel le procédé comprend une étape précédente consistant à ajouter de l'acide triphénylacétique à une solution de base libre de vilanterol dans le solvant de cétone pour former la solution de trifénatate de vilanterol dans le solvant de cétone.

7. Le procédé selon l'une quelconque des revendications 1 à 5, dans lequel le procédé comprend une étape précédente consistant à mélanger le trifénatate de vilantérol avec une quantité appropriée du solvant de cétone et à chauffer le mélange jusqu'à dissolution.

8. Le procédé selon l'une quelconque des revendications 1 à 7, dans lequel la solution du trifénatate de vilantérol est une solution saturée.

9. Le procédé selon l'une quelconque des revendications 1 à 8, comprenant en outre la recristallisation du trifénatate de vilanterol obtenu dans l'une quelconque des revendications 1 à 7 à partir d'un solvant de cétone choisi dans le groupe constitué de la méthyléthylcétone (MEK), la méthylisobutylcétone (MIK), l'éthylisopropylcétone, la méthylisopropylcétone, la 3-méthyl-2-pentanone et un mélange de ceux-ci.

10. Le procédé selon la revendication 9, dans lequel le procédé comprend l'ensemencement avec des cristaux de trifénatate de vilantérol pour initier la recristallisation.

11. Le procédé selon les revendications 9 ou 10, dans lequel la recristallisation est réalisée à partir de MEK.

12. Le procédé selon les revendications 9 ou 10, dans lequel la recristallisation est réalisée à partir de MIK.

13. Le procédé selon les revendications 1 ou 9, dans lequel la cristallisation ou la recristallisation est réalisée par mûrissement d'Ostwald.

14. Le procédé selon la revendication 13, dans lequel le mûrissement d'Ostwald comprend les étapes suivantes :
a) fournir une suspension de trifénatate de vilantérol dans le solvant de cétone ;
b) chauffer la suspension de l'étape a) jusqu'à l'obtention d'une solution ;
c) refroidir la solution à une première température supérieure à la température ambiante ;
d) ensemencer la solution avec des cristaux de trifénatate de vilantérol pour donner une suspension ;
e) refroidir la suspension à une deuxième température entre la première température et la température ambiante tout en agitant ; chauffer la suspension à la première température tout en agitant ; et répéter l'étape e) au moins une fois de plus ;
f) refroidir la suspension à une température allant de 20 °C à 25 °C ; et
g) isoler les cristaux de trifénatate de vilanterol de la suspension de l'étape f) à température ambiante.

15. Le procédé selon la revendication 14, dans lequel le solvant de cétone est MEK ou MIK.
